(19) Europäisches Patentamt
European Patent Office
Office européen des brevets

(11) Publication number : 0 252 422 B1

(12) # EUROPEAN PATENT SPECIFICATION

(45) Date of publication of patent specification : 24.07.91 Bulletin 91/30

(51) Int. Cl.$^5$ : C07D 237/04, C07D 237/08, C07D 407/12, A61K 31/50, C07D 237/14

(21) Application number : 87109469.4

(22) Date of filing : 01.07.87

(54) **Pyridazinone derivatives and salts thereof.**

The file contains technical information submitted after the application was filed and not included in this specification

(30) Priority : 07.07.86 JP 158992/86

(43) Date of publication of application : 13.01.88 Bulletin 88/02

(45) Publication of the grant of the patent : 24.07.91 Bulletin 91/30

(84) Designated Contracting States : DE FR GB IT

(56) References cited :
EP-A- 0 178 189
PATENT ABSTRACTS OF JAPAN, vol. 10, no. 56 (C-33)[2113], 6th March 1986; & JP-A-60 197 660 (MITSUBISHI KASEI KOGYO K.K.) 07-10-1985

(73) Proprietor : MITSUBISHI KASEI CORPORATION
5-2, Marunouchi 2-chome Chiyoda-ku
Tokyo 100 (JP)

(72) Inventor : Okushima, Hiromi
2747-3, Ozenji Asao-ku
Kawasaki-shi Kanagawa 215 (JP)
Inventor : Narimatsu, Akihiro
3-3, Tsutsujigaoka Midori-ku
Yokohama-shi Kanagawa 227 (JP)
Inventor : Kobayashi, Makio
1-18, Tsurukawa 2-chome
Machida-shi Tokyo 194 (JP)
Inventor : Furuya, Rikizo
12-1, Naruse 2-chome
Machida-shi Tokyo 194 (JP)
Inventor : Kitada, Yoshimi
6-20, Satsukigaoka Midori-ku
Yokohama-shi Kanagawa 227 (JP)
Inventor : Tsuda, Kunio
103 Seiwa-Manshon 4-18-24 Soshigaya
Setagaya-ku Tokyo 157 (JP)

(74) Representative : TER MEER - MÜLLER - STEINMEISTER & PARTNER
Mauerkircherstrasse 45
W-8000 München 80 (DE)

EP 0 252 422 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to novel pyridazinone derivatives and salts thereof.

Hitherto, various cardiotonics having a pharmaceutical activity for directly strengthening the contractile force of heart have been utilized for the cure of heart failure.

However, many of the cardiotonics have disadvantages such as tendency to cause arrhythmia due to their extremely narrow range of safety dosage, transient cardiotonic activity and unsuitability for oral administration. EP-A-0.178189 and JP-A-60-197660 disclose pyridazinone derivatives with a cardiotonic activity.

We have sought for a compound having high cardiotonic activity and capable of exhibiting sufficiently long lasting activity, and we are now discovered the pyridazinone derivatives of the present invention.

It is therefore an object of the present invention to provide novel pyridazinone derivatives and salts thereof which are useful as cardiotonics having high and long lasting activity.

The pyridazinone derivatives of the present invention are best described by reference to the following general formula Ia ;

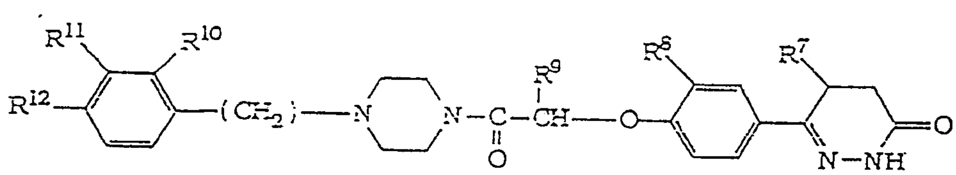

(Ia)

wherein $R^7$ represents a hydrogen atom or a $C_1$-$C_5$ alkyl group, $R^8$ represents a hydrogen atom or a $C_1$-$C_5$ alkoxy group, $R^9$ represents a hydrogen atom or a $C_1$-$C_5$ alkyl group, each of $R^{10}$ and $R^{11}$ represents a hydrogen atom or a $C_1$-$C_5$ alkoxy group independently and $R^{12}$ represents a $C_1$-$C_5$ alkoxy group.

In the general formula I, preferable examples of $R^7$ and $R^9$ includes methyl, ethyl and propyl group ; preferable examples of $R^8$ include methoxy, ethoxy and propoxy group.

Preferable examples of $R^{10}$, $R^{11}$ and $R^{12}$ include methoxy, ethoxy and propoxy group. Preferred compounds of formula Ia are those wherein $R^7$ represents an hydrogen atom.

Examples of the pyridazinone derivatives of the general formula I are shown below :

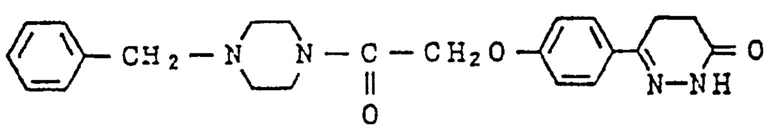

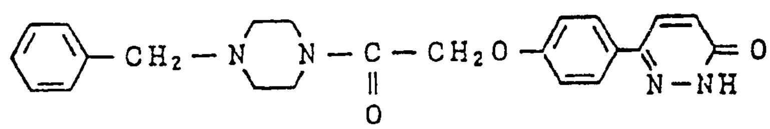

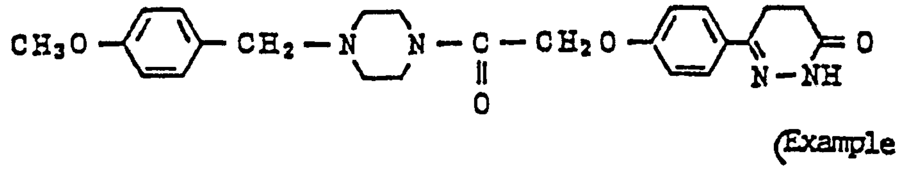

(Example' 3 )

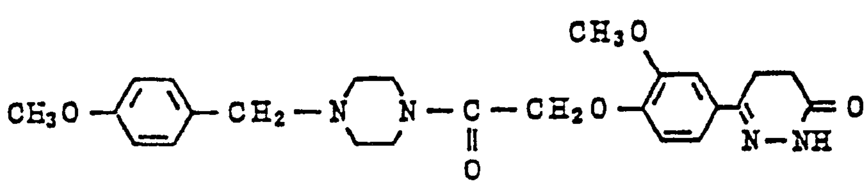

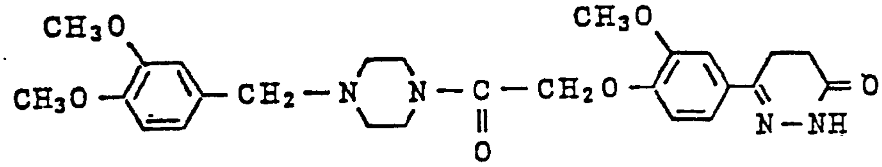

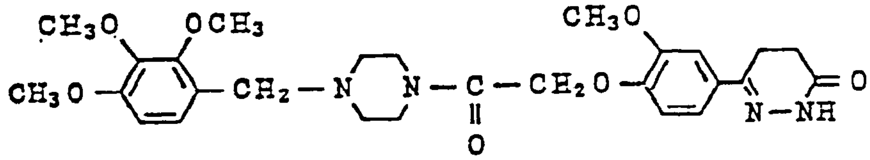

(Example 1)

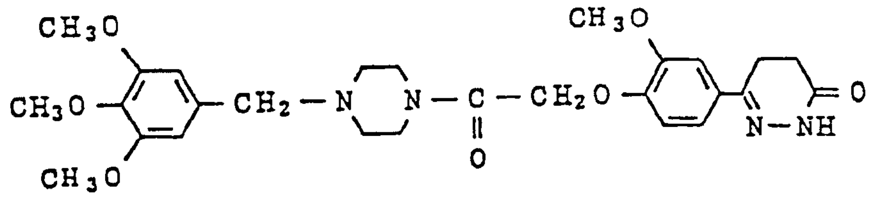

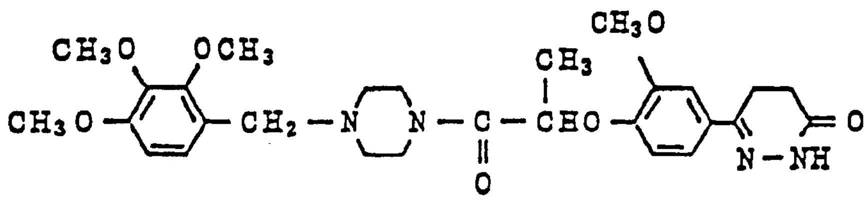

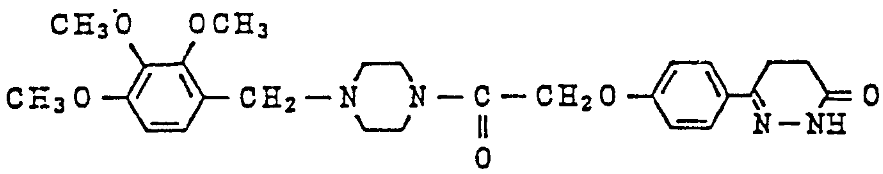

(Example 2)

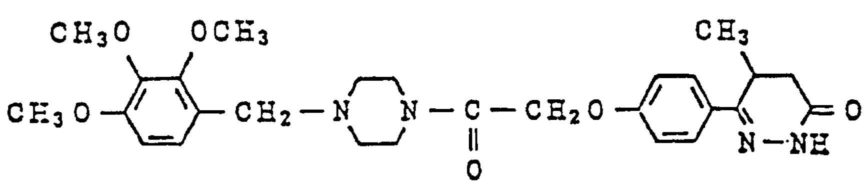

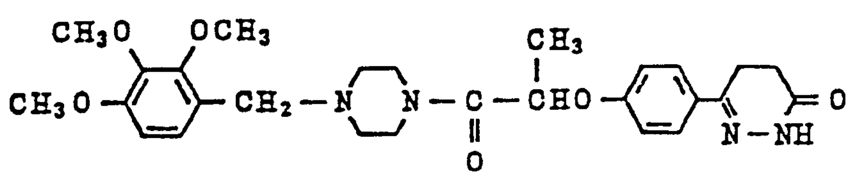

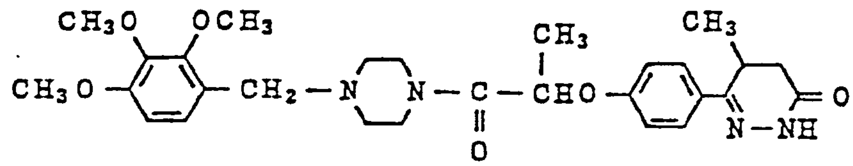

Pharmaceutically acceptable salts of the above-mentioned compounds are also included within the scope of the invention. Examples of the salt include a salt of mineral acid such as hydrochloric acid and phosphoric acid and a salt of organic acid such as lactic acid and butyric acid.

A process for the preparation of the compounds according to the invention will be illustrated below.

The pyridazinone derivatives of the general formula I can be prepared, for example, by the following route.

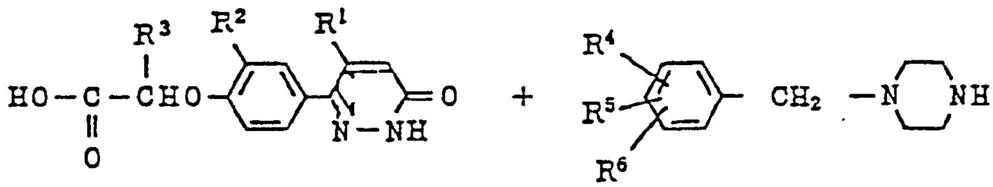

(II) (III)

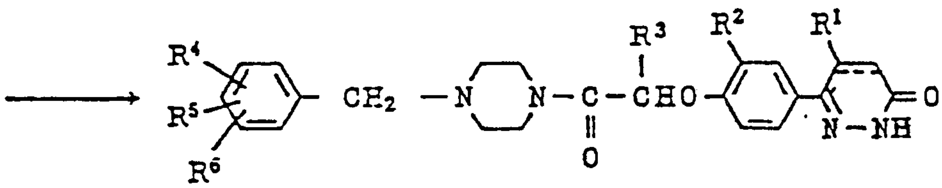

(I)

The starting material of carboxylic acid represented by the general formura II can be prepared by the following route ;

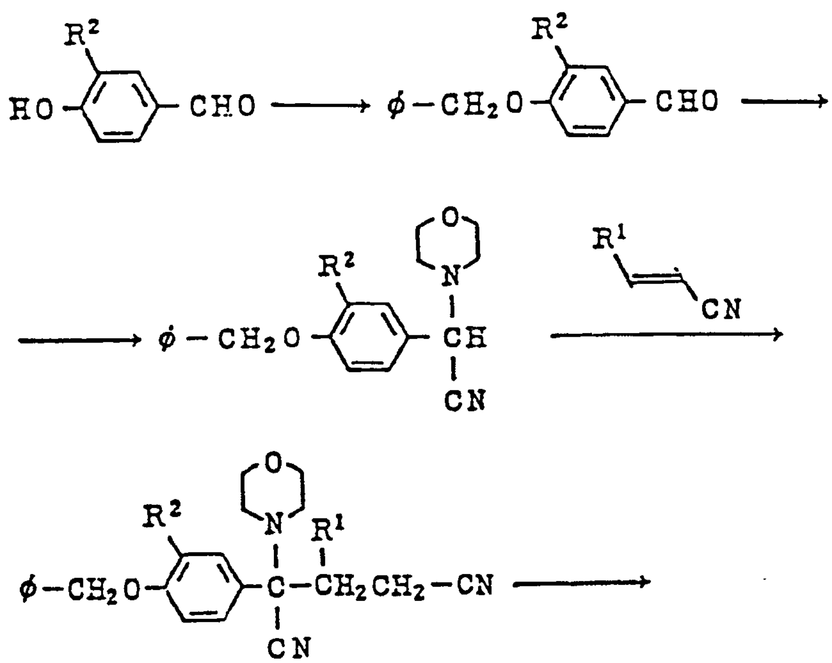

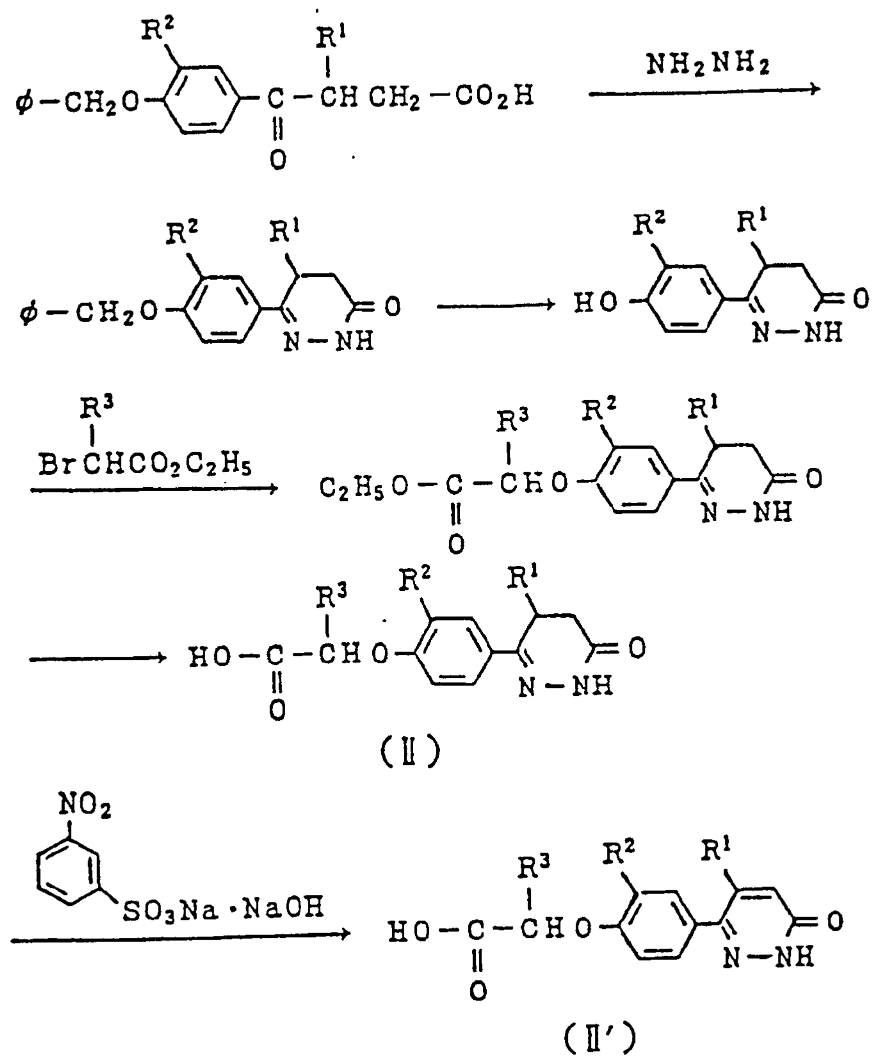


(II)

(II')

wherein $R^1$, $R^2$, $R^3$, $R^4$, $R^5$, $R^6$ have the same meanings as already defined in the general formula Ia.

Thus, the compound of general formula I can be prepared by a reaction forming an amide coupling between the carboxylic acid represented by the general formula II and the amine represented by the general formula III.

The reaction forming the amide coupling can be taken place, for example, by (i) reacting carboxylic acid (II) with alkyl halocarboxylate to form a mixed acid anhydride, which is then reacted with amine (III) (mixed acid anhydride method), (ii) condensating carboxylic acid (II) and amine (III) under the presence of a dehydrating agent such as dicyclohexyl-carbodiimide (carbodiimide method) and (iii) any other suitable method such as carboxylic acid halide method and active ester method. Among these methods, the mixed acid anhydride method is particularly preferred.

The mixed acid anhydride is prepared by reacting a carboxilic acid (II) with an alkyl halocarboxylate under the presence of a basic compound, for example, organic base such as triethylamine, pyridine and diazabicycloundecene, and mineral base such as potassium carbonate, in a solvent or a mixture of solvents conventionally used for the mixed acid anhydride method. Examples of the solvent include tetrahydrofuran, dioxane, toluene, chloroform, ethyl acetate, dimethylformamide, dimethylacetamide and the like. The reaction temperature is about from $-20$ to 100°C and the reaction time is about 5 to 10 hours.

Generally, the obtained mixed acid anhydride can be directly reacted with the amine (III) without further isolation. The reaction can be carried out at $-20$ to 100°C for about 5 minutes to 10 hours. The amine (III) is a well known compound.

The compound according to the invention can be administered as a cardiotonic orally or parenterally in a suitable form.

For example, the compound of the invention can be made in the form of powder, granule, tablet, dragee, pill, capsule and solution for oral administration, and it can be made in the form of suppository, suspention,

solution, emulsion, ampour, injection solution for parenteral administration. A combination of them may of course be provided. The compound of the present invention can be made into pharmaceutical composition by any conventional method well known in the art.

The administration dose of the compound of the present invention may be decided by a physician according to the age, sex, body weight, sensibility, degree of disease, physical condition of a patient to be treated, method, time and interval of administration, pharmaceutical property, kind of formulation form and kind of active ingredient of the pharmaceutical composition etc.

For example, a daily dose of the compound is selected in the range of 0.1 to 10 mg/kg for oral administration, but it is of course not limited within it.

The invention will be further illustrated hereinafter by way of examples.

Example 1 : Preparation of ;

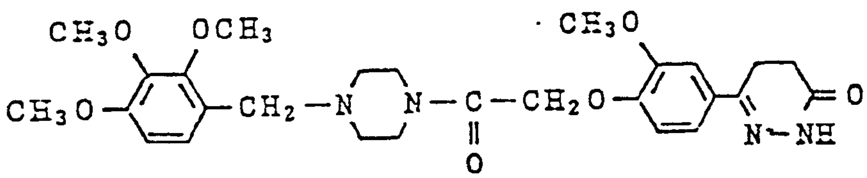

Into 5 ml of N,N-dimethylformamide and 7 ml of tetrahydrofuran, 0.22 g of

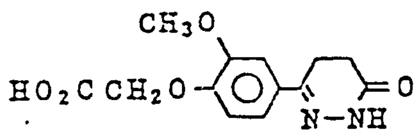

was dissolved, and the solution was cooled to – 20 to –30°C. Then, the solution was added with 0.11 ml of triethylamine and 0.076 ml of ethyl chloroformate succesively to form a mixed acid anhydride.

The cold reacticn mixture was added with 0.22 g of

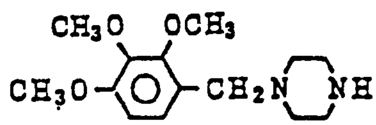

and left at –20°C for 20 minutes, and then gradually warmed to the room temperature and allowed to react for 2 hours. The reaction mixture was concentrated and chromatographed on silica gel column (solvent : chloroform → chloroform/methanol = 30/1) to isolate the object product in oily form.

The oily product was dissolved in 10 ml of ethanol and added with 1.5 ml of 1N-HCl/ethanol and 100 ml of ether successively to precipitate a hydrochloride salt of the object compound. The precipitated solid was filtered out, washed with ether and dried to obtain a white solid of hydrochloride salt of the object compound.

| | |
|---|---|
| Yield : | 0.33 g ; |
| melting point : | 138.0-140.0°C ; |
| IR : | 1670 $cm^{-1}$ |

Example 2 : Preparation of ;

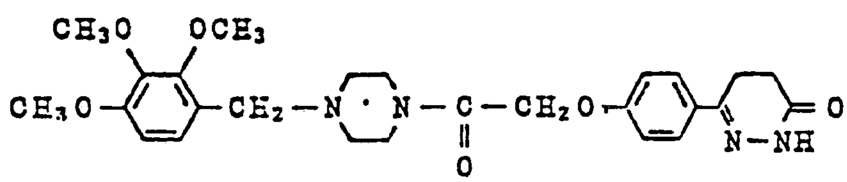

Into 5 ml of N,N-dimethylformamide and 7 ml of tetrahydrofuran, 0.20 g of

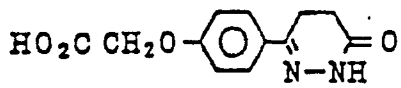

was dissolved, and the solution was cooled to – 20 to –30°C. Then, the solution was added with 0.11 ml of triethylamine and 0.076 ml of ethyl chloroformate succesively to form a mixed acid anhydride.

The cold reaction mixture was added with 0.22 g of

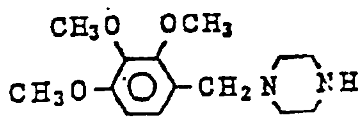

and left at –20°C for 20 minutes, and then gradually warmed to the room temperature and allowed to react for 2 hours. The reaction mixture was concentrated and chromatographed on silica gel column (solvent : chloroform → chloroform/methanol = 30/1) to isolate the object product in oily form.

The oily product was dissolved in 10 ml of ethanol and added with 1.5 ml of 1N-HCl/ethanol and 100 ml of ether successively to precipitate a hydrochloride salt of the object compound. The precipitated solid was filtered out, washed with ether and dried to obtain a white solid of hydrochloride salt of the object compound.

| | |
|---|---|
| Yield : | 0.34 g ; |
| melting point : | 215.0-216.0°C ; |
| IR : | 1675 $cm^{-1}$ |

<u>Example 3 : Preparation of</u> ;

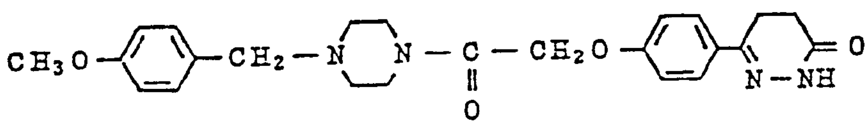

Into 4 ml of N,N-dimethylformamide and 6 ml of tetrahydrofuran, 0.25 g of

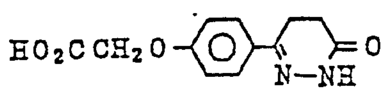

was dissolved, and the solution was cooled to – 20 to –30°C. Then, the solution was added With 0.14 ml of triethylamine and 0.095 ml of ethyl chloroformate successively to form a mixed acid anhydride.

The cold reaction mixture was added with 0.22 g of

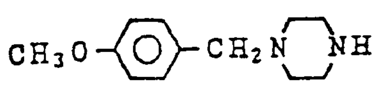

and left at –20°C for 20 minutes, and then gradually warmed to the room temperature and allowed to react for 2 hours. The reaction mixture was concentrated and chromatographed on silica gel column (solvent : chloroform → chloroform/methanol = 20/1) to isolate the object product in oily form.

The oily product was dissolved in 20 ml of ethanol and added with 1.5 ml of 1N-HCl/ethanol and 100 ml of ether successively to precipitate a hydrochloride salt of the object compound. The precipitated solid was filtered out, washed with ether and dried to obtain a white solid of hydrochloride salt of the object compound.

| | |
|---|---|
| Yield : | 0.41 g ; |
| melting point : | 239.0-240.5°C (decomposition) ; |
| IR : | 1670 $cm^{-1}$ |

Example 4

The usefulness of the compounds obtained in Examples 1 to 3 as a cardiotonic is determined by means of the standard pharmacological test methods. For example, the activity of the compounds is demonstrated by determining an increase of the contractile force of isolated dog papillary muscle and isolated guinea pig left atrium muscle, as well as an increase of the cardiac contractile force of anesthetized dog, all of which are caused by the compounds. The pharmacological test methods will be described below.

1) Method of using cross-circulated preparation of isolated dog papillary muscle

Cross-circulated preparations of isolated dog papillary muscle were prepared according to the method of Endoh & Hashimoto, American J. Physiol., vol. 218, p.1459-1463, 1970, United States. The compound dissolved in solvent was injected to the artery close to the preparation, and the action of the compound on the contractile force of papillary muscle was determined.

2) Method of using isolated guinea pig left atrium

Male guinea pips weighing 200-300 g were killed by hitting their back of the heat and their left atria were immediately isolated. The left atrium was fixed to the bottom of an organ bath filled with 30 ml of Klebs-Henseleit's solution maintained at 35°C. A mixed gas comprising 95% of $O_2$ and 5% of $CO_2$ was passed through the Klebs-Henseleit's solution in the organ bath. The auricle of the left atrium was connected to a transducer with a thread to measure the isometric contraction. The atrium was applied with 0.5 g of resting tension. The atrium was electrically driven by square wave of 1 millisecond duration at a voltage of the order of 1.5 times of the threshold voltage and at a rate of twice per second by means of two platinum electrodes. The preparation was stabilized for 30 minutes, then the compound dissolved in solvent was added to the organ bath, and the reaction was determined.

3) Method of using anesthetized dog

Male or female mongrel dogs weighing 8-15 kg were used. The dogs were anesthetized with sodium pentobarbital at the dose of 30 mg/kg (intravenous injection) and then applied with artificial respiration. They were thoracotomised at the intercostal portion between forth and fifith costa, and the fifth costa was excised. Then the pericardium was cut to expose the heart. A probe of an electromagnetic flow meter was attached to the aorta to measure the blood flow rate in the aorta, which was used as an approximate index of the cardiac output (CO). A polyethylene cannula was inserted into the left cardiac ventricle to measure the intraventricular pressure, thereby the variation rate of the intraventricular pressure of the left cardiac ventricle (dp/dt) was electrically determined. A strain gauge was attached to the wall of the right cardiac ventricle to measure the contractile force of the right cardiac ventricle muscle (Cont). The systemic blood pressure was measured at the left femoral artery. The heart rate was measured by means of a cardiotachometer. The compound dissolved in solvent was applied intravenously from the left femoral vein.

In the pharmacological tests described above, all of the cardiotonics according to the invention increased the contractile force of dog papillary muscle and guinea pig left atrial muscle. They also increased the maximum rate of rise in the intraventricular pressure of the left cardiac ventricle (dp/dt max), Cont and CO in anesthetized dogs, that is, they increased the cardiac contractility.

The increasing rate of the contractile force of dog papillary muscle and guinea pig left atrium, as well as the increasing rate of dp/dt max, Cont and CO of anesthetized dog are shown in Table 1 below.

From the results shown in Table 1, the pyridazinone derivatives according to the invention are clearly excellent cardiotonics having high cardiotonic activity.

Table 1

| Compound | Contractile force of dog papillary muscle | | Contractile foece of guinea pig left atrium | | Anesthetized dog | | | |
|---|---|---|---|---|---|---|---|---|
| | Dosage (μg i.a.) | Increasing rate (%) | Dosage (g/ml) | Increase rate (%) | Dosage (μg/kg i.v.) | dp/dtmax Increasing rate (%) | Cont Increasing rate (%) | Co Increasing rate (%) |
| Compound of Example 1 | 30 | 10.0 | $10^{-5}$ | 35.5 | 30 | 12.9 | 7.9 | 5.5 |
| | 100 | 20.1 | $3x10^{-5}$ | 44.8 | 100 | 27.1 | 21.5 | 13.3 |
| Compound of Example 2 | 30 | 11.4 | $10^{-5}$ | 6.4 | 30 | 12.2 | 10.1 | 4.8 |
| | 100 | 12.5 | $3x10^{-5}$ | 22.6 | 100 | 34.3 | 22.8 | 16.3 |
| Compound of Example 3 | 30 | 13.2 | $10^{-5}$ | 32.0 | 30 | 18.0 | 12.2 | 17.4 |
| | 100 | 17.0 | $3x10^{-5}$ | 28.0 | 100 | 49.8 | 29.1 | 28.9 |

## Claims

1. A pyridazinone derivative represented by the following formula Ia :

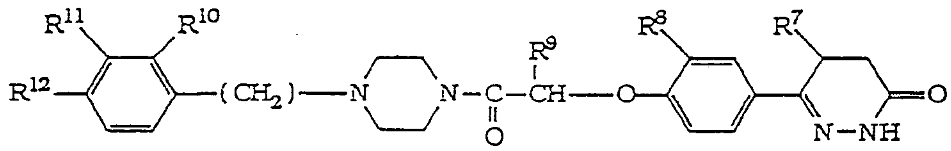

(Ia)

wherein $R^7$ represents a hydrogen atom or a $C_1$-$C_5$ alkyl group, $R^8$ represents a hydrogen atom or a $C_1$-$C_5$ alkoxy group, $R^9$ represents a hydrogen atom or a $C_1$-$C_5$ alkyl group, each of $R^{10}$ and $R^{11}$ represents a hydrogen atom or a $C_1$-$C_5$ alkoxy group independently and $R^{12}$ represents a $C_1$-$C_5$ alkoxy group.

2. The compound as defined in claim 1 wherein $R^7$ represents a hydrogen atom.

## Patentansprüche

1. Pyridazinonderivat der allgemeinen Formel Ia

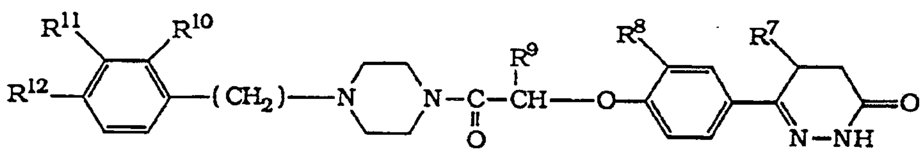

(Ia)

in der $R^7$ ein Wasserstoffatom oder eine $C_1$-$C_5$-Alkylgruppe, $R^8$ ein Wasserstoffatom oder eine $C_1$-$C_5$-Alkoxygruppe, $R^9$ ein Wasserstoffatom oder eine $C_1$-$C_5$-Alkylgruppe, $R^{10}$ und $R^{11}$ jeweils unabhängig voneinander Wasserstoffatome oder $C_1$-$C_5$-Alkoxygruppen und $R^{12}$ eine $C_1$-$C_5$-Alkoxygruppe bedeuten.

2. Verbindung nach Anspruch 1, worin $R^7$ ein Wasserstoffatom bedeutet.

## Revendications

1. Dérivé de pyridazinone représenté par la formule Ia ci-dessous :

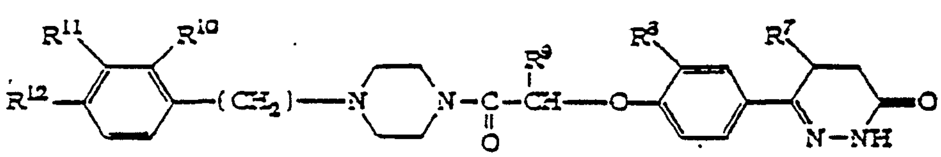

(Ia)

dans lequel $R^7$ représente un atome d'hydrogène ou un groupe alkyle en $C_1$-$C_5$, $R^8$ représente un atome d'hydrogène ou un groupe alkoxy en $C_1$-$C_5$, $R^9$ représente un atome d'hydrogène ou un groupe alkyle en $C_1$-$C_5$, $R^{10}$ et $R^{11}$ représentent indépendamment un atome d'hydrogène ou un groupe alkoxy en $C_1$-$C_5$, et $R^{12}$ représente un groupe alkoxy en $C_1$-$C_5$.

2. Le composé défini dans la revendication 1 dans lequel $R^7$ représente un atome d'hydrogène.